# EUROPEAN PATENT APPLICATION

(11) **EP 4 220 534 A1**
(43) Date of publication of application: **02.08.2023**
(21) Application number: 21877924.7
(22) Date of filing: 02.10.2021
(51) Int. Cl.: G06Q 50/26, G06K 19/06, G16H 50/80, G06Q 50/12

(54) **INTEGRATED MANAGEMENT METHOD FOR PREVENTING SPREAD OF CONTAGIOUS DISEASES**

(30) Priority: 05.10.2020 KR 20200128703
(71) Applicant: Agaram.Com, Ltd., Seoul 01885 (KR)
(72) Inventor: JO, Wangje, Seoul 03064 (KR)
(74) Representative: Sach, Greg Robert
(86) International application number: PCT/KR2021/013544
(87) International publication number: WO 2022/075672

(57) **Abstract**

The world is currently facing serious danger due to COVID - 19 , even Korea , where is stated a good quarantine system , is confronting a serious risk of spread despite of a single group infection occurs. Taking this situation into consideration, it is intended to build a system that can check the spread path in real time. The business s print the bar code and attached it to the table after registering the information. Customers scan the attached bar code to the table through the downloaded app to register for entry when they visit the business s. If customers register their leave in the app, the date and time of leave will be recorded. When an infected person occurs, the customer's mobile phone number and visiting period will be searched and immediately extracted business s and customers with a potential for infection. Therefore, quarantine can be carried out.

## Description

### DETAILED DESCRIPTION OF THE INVENTION

### Purpose of Invention

### Field of Invention

The present invention relates to a method for efficiently controlling the spread by establishing a system that can check the infection route of a spreading contagious disease (currently COVID - 19) in real time.

### Background of Invention and Prior Technology

The world is currently facing serious danger due to COVID - 19, even Korea, where is stated a good quarantine system, is confronting a serious risk of spread despite of a single group infection occurs. In addition, it is important to have an effective method to prevent the spread due to contagiousness, but until now, a method of collectively controlling regions or industries has been used.

This has a serious negative impact on the overall economy, but the effect is short-term, and it has a problem that it can spread again at any time even after it is dealt with. Here, in the process of tracing the movement of an infected person and performing an inspection to prevent further spread, there is a problem that a lot of cost and time are required, and even inaccuracies occur as almost all tasks are performed by humans.

In addition, as a system for confirming the existing route of infection, all customers who visit the business scan the pre-registered QR code with a reader provided at the business, or customers without a QR code are asked to manually record their customer information. However, in reality, customer information is written down by hand as most of the customers do not have a QR code and the problem of having to have a separate reader and the inconvenience of use.

Here, the handwritten ledger brings an leakage of personal information which is a serious problem, and possibly people can access records as handwritten records and QR at the same time, when identifying the actual movement of the infected person. Therefore, it is necessary to check one by one, and also the leave time is unclear. After the visiting of the infected person, there is a problem of conducting a test and quarantine for 2 weeks for everyone. In other words, it is understandable from the viewpoint that even a small risk cannot be taken from a conservative standpoint, but the quarantine measures have serious personal and economic damage, human rights violations, and excessive inspection costs. As a result, an effective method considering appropriate risks and costs is required.

### Technical task to be achieved by the invention

The present invention is to build a system that conveniently records possible contagious movements using mobile phones owned by most of the people, and effectively extracts information on people who have been in contact with confirmed infected people to take quarantine measures.

### Composition and operation of the invention

In order to achieve the above object, downloading and installation a solution are required to operates on a necessary PC or smart phone after registering in the integrated system in advance for almost all restaurants used by an unspecified number of people, which is the main route of transmission, print or purchase information (QR and 1 or 2 dimensional bar codes, numbers, etc.) and attach them to the entrance and tables of the business.

When customers visit the store, they turn on the pre-installed smart phone app and recognize the identification information through a camera or key input that is attached to the entrance or table, recording the entry date and time and information about the table in real time. When customers make a payment, the date and time of departure shall be recorded. Here, if necessary, the number of customers registered in the table and the registered number can be checked in real time, and in case of discrepancies, all of them can be asked to register.

In addition, it is necessary to obtain identification information at all places, including restaurants with takeout and other places where there is a possibility of infection, such as classrooms at universities and academies, theaters, taxis, and religious facilities such as churches where people gather, to record attendance, In the case of a mobile phone that unable to use the app, entry and leave can be recorded using the caller ID when calling the phone number registered by the business.

Furthermore, if a record is made, information (image, text, etc.) that registered and transmitted to the customer's mobile phone, and businesses centered on take-out that unused tables can control so that no customers are missed by checking the received information.

Afterwards, if you enter the cell phone number of the infected customer and the date and time of possible infection, you can check the cell phone number and table information of the business s and customer with the possibility of infection. By extracting this high customer, you can proceed with the inspection and live.

Here, the possibility of infection can be determined by evaluating the risk level considering the time zone for contact with the infected person and the distance of the table to determine the subject and order of examination, and if necessary, it will be possible to recommend adjusting the arrangement and distance of the table.
In actual application, the techniques described above will be appropriately combined and used to suit the situation, and the method and apparatus according to the present invention will be described in detail with reference to the accompanying drawings.

The examples herein are provided for illustrative purposes only and not to limit the present invention, and although the present invention has been described with reference to exemplary embodiments, the terms used herein are intended to be explanatory or illustrative rather than limiting. It should be understood that it is used for

In addition, various modifications and variations within the scope of the appended claims do not depart from the spirit and scope of the present invention.
definitions may be made, and the present invention has been described herein with reference to exemplary embodiments based on specific systems, the present invention is not limited thereto and may be extended to functionally equivalent structures, methods, and uses within the scope of the appended claims. there is

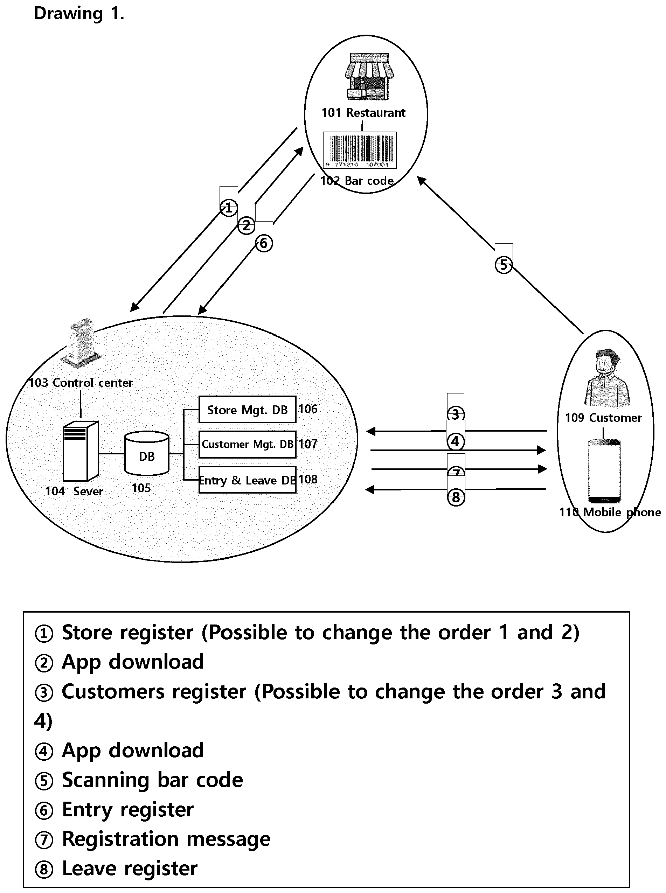

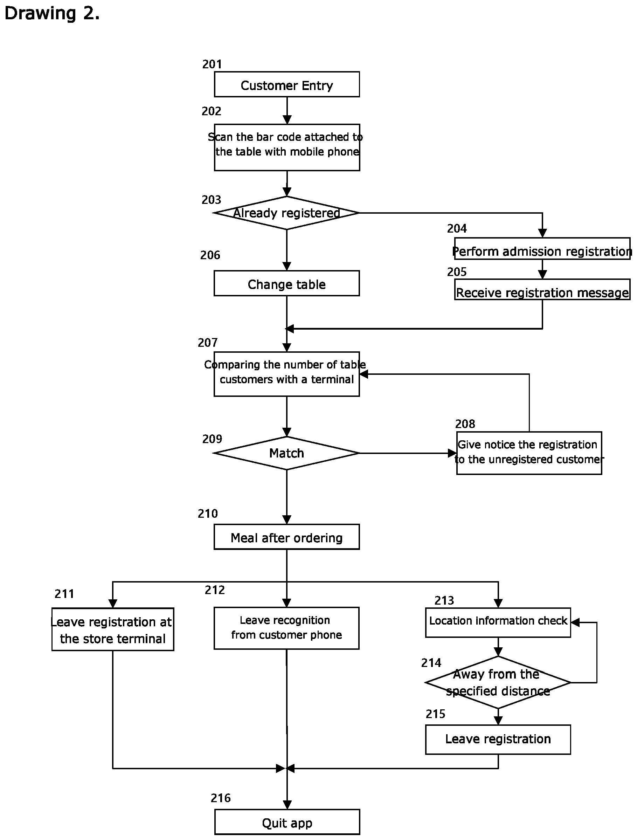

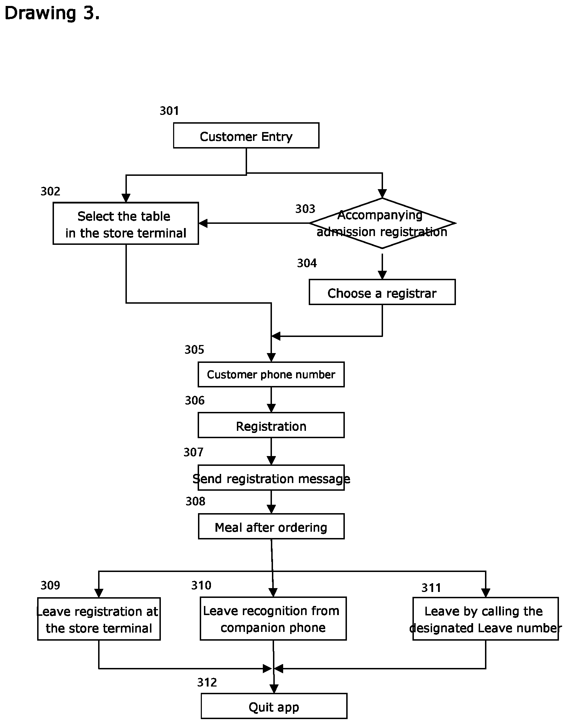

Figure 1 is a general flowchart of the present invention. Businesses register business s information through online and offline (106), download the app, or download the app and register the business s through the app (106), then print the assigned barcode. and attach it to the table. And after the customer downloads the app, agrees to the basic information and completes the registration (107), then it becomes available for use. When a customer visits the business (101) , turns on the app, and scans the barcode (102) attached to the table, the business s information and the date and time of entry are immediately recorded (108) . (108) will be.

Here, since customer registration is to prevent the spread of contagious disease, it is reasonable not to collect any information that may invade customers' privacy, so customers are classified only by mobile phone number, and route information is the minimum specified for quarantine. It is reasonable to destroy it immediately after the period of time has elapsed. If sensitive personal information about movement is used in violation of these principles, it will worsen a situation of serious confusion in which customers will never cooperate with quarantine. However, the information of the customer whose infection has been confirmed can be further inquired by using the telecommunications company and other information.

Drawing 2 is a flowchart of entry and leaveof a restaurant with a table. When a customer enters and is guided to a table, the customer scans the barcode attached to the table after turning on the app (202) , and if not registered, the control center registers the entry ( 204) is performed, and the mobile phone receives a registration completion message (205) , and if it is already registered, it changes the table information ( 206 ) . Here, the establishment checks (207) the number of customers in the table and the actual number of customers through the terminal, and if there is a difference, induces (208) to register unregistered customers.

Here, when searching for the registered mobile phone number in the table from the terminal of the establishment, only the last 4 digits are opened and checked, but the entire number is not opened, which conforms to the purpose of protecting personal information.

And if the customer places an order and finishes the meal (210) and leaves, the leaveregistration is performed (212) through the app, and the business s can also register the customer at the table to leave (211) , but whichever is done first In one case, it is natural that the following is unnecessary . However, if the customer and the business s do not register to exit, the current location of the customer and the location of the business s are compared (213), and if the location is more than a certain distance away (214), leaveregistration (215) is automatically performed.

Here, the app will be possible in principle on the premise that it will not be terminated while registered for entry into the establishment and will operate in the background, and since these functions and implementations are already known technologies, they will not be discussed separately here. And, as defined below, the barcodes 102 and 202 can be all numbers, texts, images, and loT that can receive the necessary codes from a mobile phone.

Of course, if there is information on a business s that has not been exited from the time of business s registration, it is natural to register the leaveautomatically, and in the case of a university lecture room, set the lecture hall itself to be recognized as a table, and then set the student (customer) is configured to additionally input the seat number.

In addition, it will be possible to manage temporary public transportation such as taxis, buses, and subways by attaching barcodes to each vehicle , and it will be possible to manage small and medium-sized businesses , hospitals , and beauty salons with only one table (00) , and various other industries and It will be possible to include all establishments across the country within the quarantine network by modifying and using it in establishments . Here , since it may be difficult to apply 1.2 -dimensional barcodes to buses and subways due to congestion during rush hour, the same information as 1- and 2 -dimensional barcodes can be transmitted to mobile phones using the Internet of Things device mentioned in the definition of barcodes , and received If you leave the distance, you will be dismissed .

Drawing 3 is an entry and leaveflow chart when there is no app-enabled mobile phone. If the customer accompanied by the customer registers first after entering (303) , select the business s (304) and enter the customer's mobile phone number to add (305 ) , and if there is no registered customer or if you use the terminal of the establishment from the beginning, select the table (302) and enter the mobile phone number of the customer to be added (305) , the entry registration is completed (306) , and the system Sends (307) a registration message to the number .
, when the customer places an order and finishes the meal (308) and leaves, the leaveregistration is performed through the accompanying app (310) , and the caller ID is used when calling the designated number for leaveregistration sent with the registration message. You can register (311) to leave, and the business can also register (309) to leave the customer from the table, but if either one did it first, it is natural that the following is unnecessary.

Here, the case where the customer does not have a cell phone number may be a case of infants and young people, or a country or region where mobile phones are not widely distributed. In most cases, infants and teenagers live together with their parents, so they can be added by entering "001∼009" instead of "010", which is the classification code of the mobile phone. In addition, since the classification code of a mobile phone is different for each country, it can be modified according to the characteristics of the country, so the above example is for reference only.
will be able to register by entering information that can identify the individual, such as the social security number (resident registration number in Korea) given to citizens by the country. For infants and young people, social security numbers may be used for individual management.

The registration message can provide a unique number that can be used when exiting, where the unique number is registered and used as many numbers as necessary in the system unit, so there is nothing to bear on the business s because it is not assigned a number for each establishment. This will be a much more efficient method than having to have a number for each establishment, which was previously applied. In addition, the method of extracting and using the actual caller ID is a known technology, so it is not discussed separately here.

In addition, if the mobile phone number is not a normal mobile phone number, it is natural that the registration message is not sent and it is impossible to leaveusing the caller ID. In addition, even if it is a normal mobile phone number, sending a registration message is not an essential requirement of this patent. In other words, depending on the configuration method of the system, whether or not to send may be considered.

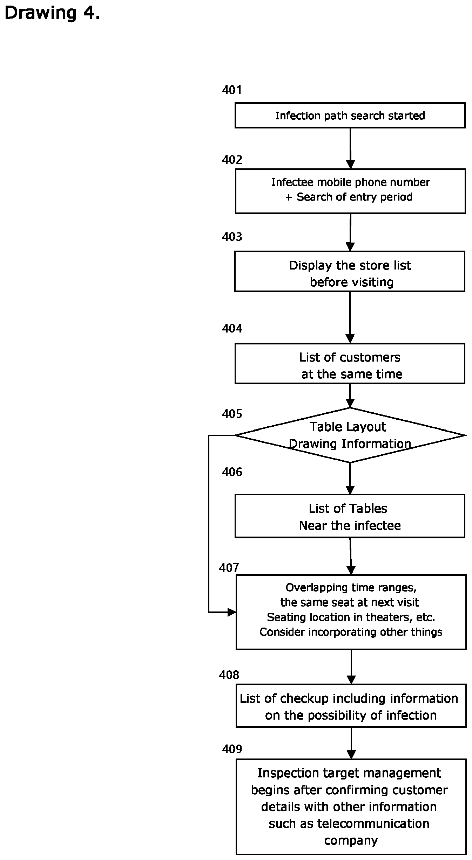

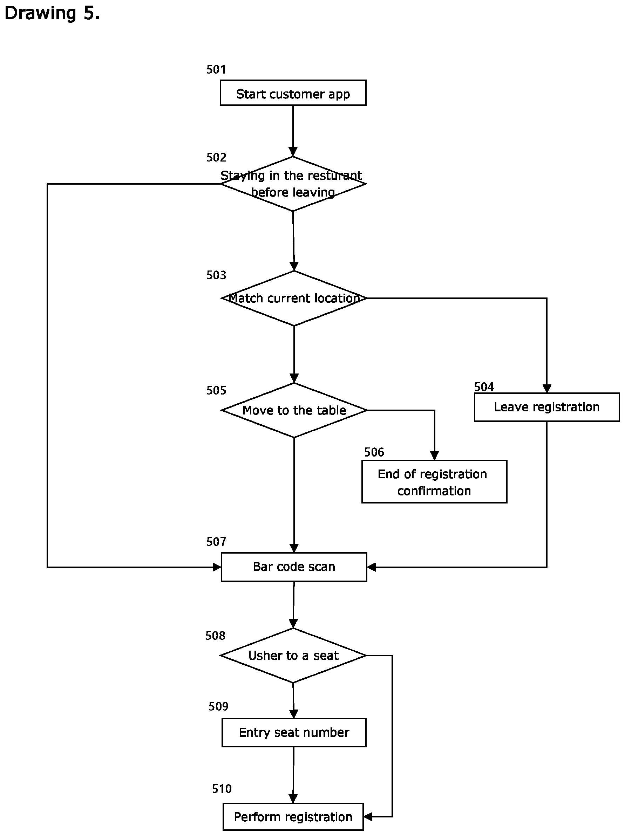

Figure 4 is a flow chart for extracting the infected person's path and inspection target. If you enter the infected person's mobile phone number and search period (402) and inquire, you can query (403) all lists of businesses visited during the applicable period, If you select the establishment, you can search the list of customers from the same time (404) , and if there is additional information (405) on the table arrangement drawing from the establishment, you can check the table used by the infected person and information on the surrounding tables (406). there is
you can check the list (407) considering the degree of infection risk for all customers of the target establishment by using the overlapping time zone , table location , customers after the infected person used the table , seat location in the case of a theater, and other business s information , After confirming the customer's detailed information (409) with other information such as the telecommunications company, appropriate inspection and quarantine measures can be taken according to the degree of risk .

the target into urgent inspection, normal inspection, and postponement of inspection through detailed information, so that information can be efficiently carried out. You can set it to extract. In addition, it will be relatively helpful for economic activity as not all customers are isolated according to grade.

In particular, seating arrangements in classrooms are important for universities and academies that do not have fixed seating arrangements, and if this information can be confirmed, information on emergency quarantine targets can be extracted very efficiently.

And since the protection of personal information is very important, "In the process of checking the business s visit record, the quarantine authorities only partially display the cell phone number, and only display the entire number when it is subject to final quarantine". As such, it is very important to set up and implement thorough regulations regarding access to personal information.

Drawing 5 is a flowchart of the operation method of the mobile phone app when a customer enters. The moment you turn it on, check whether there is business s information before leaving (502), if it is not the current business s (503), leaveregistration (504), and if it is the current business s If the position has not been moved (505), the confirmation is completed (506), and if the position has been moved, the barcode on the table is scanned (507). However, in the case of a lecture hall or a theater, registration is performed (510) after automatically entering seats (509).

Of course, if you want to move a seat in a classroom or theater, you can register by changing the seat in the app, and the procedure or method can be modified for customer convenience and information confirmation efficiency in the development and use of other apps.

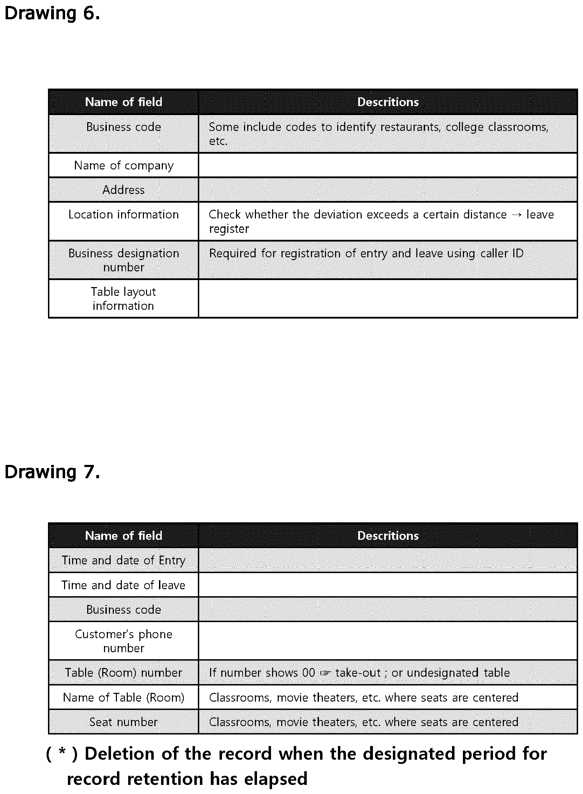

Figure 6 is a DB configuration diagram for managing business s information. The business s code includes a partial code that can distinguish the type of business, which can be set as 0 for general, 9 for lecture halls and theaters from universities, and location The information is to check the location information in the app even if the customer's leaveis not separate, and to register the leaveif the distance from the business s is more than specified, and the business s designated number is to manage entry and leaveby using the caller ID. However, the designation number is information that has nothing to do with the terminal to be used in the establishment, because there is no limit on the type and number of terminals that can be used in the establishment.
, the table arrangement information of the establishment is detailed information that determines the degree of contact with the infected person, and plays an important role in the grade of the inspection target. If necessary, it may be recommended that the arrangement be effective for quarantine or to remove some tables.

Figure 7 is a DB configuration diagram that manages customer route information. Table ( Room ) No designates a table in the case of a business s , a lecture room or a theater room, and a table ( room ) name and seat No are assigned to a university lecture room or a movie theater theater. It is set for management convenience.

In addition, in the case of Takeout, when a certain amount of time (30 minutes, etc.) has elapsed, it can be automatically removed or referred to when checking the infection route. Of course, it is most accurate for individual customers to register to leave, but it can be used incidentally if no .

In addition, since the DB configuration of Figures 6 and 7 is not fixed, and the DB and Table can be designed separately according to the characteristics of the industry, what is shown here is used for explanation or example with minimal settings. you will have to understand

### Effects of the Invention

the country participate without omission on the basis of ensuring that target businesses and customers can use them conveniently while minimizing construction costs. In addition, appropriate and efficient control measures are needed in that if strong movement control or economic activity is suppressed to prevent the spread of contagious disease, society can proceed into a serious panic. In other words, it is very important to enable economic activities while controlling appropriate risks in a situation where it is impossible to predict when the current Corona 19 (including other contagious disease) will end. This patent was designed with these points in full consideration.

### Drawing

Figure 1 is a general flow chart of the present invention
Drawing 2 is a flow chart of entry and leaveof restaurants with tables, etc.
Drawing 3 is a flow chart of entry and leavewhen there is no app-enabled mobile phone
Figure 4 is a flow chart for extracting the infected person's path and test target
Drawing 5 is a flow chart of the operating method of the mobile phone app when entering the customer
Figure 6 is a DB configuration diagram for managing business s information
Drawing 7 is a DB configuration diagram that manages customer route information

### < Description of symbols for main parts of drawings >

### 102 barcode

2 -dimensional barcodes, QR codes, image codes, and loT devices using wireless communication that can deliver information such as takeout (No. 00) and table numbers to smartphones. In addition, 1 and 2 -dimensional barcodes and QR codes are open so that they can be printed out and used from any time if necessary.

### 207 Terminal

All equipment that can run a program that can query and modify server information with similar devices, including PCs, smartphones, and pads that are connected to the Internet as devices used in the business, and there is no limit to the number of devices used in principle.

## Claims

1. The methods of preventing the spread of contagious diseases ; A control center that manages business information, customer information, and access information; Bar code linked with business and table information, Business terminals that register and inquire customers' access, A terminal for quarantine authorities to inquire the route of infection, Customer terminal used for access registration, including
Step of the inputting bar code information from the customer's terminal to transmit to the control center ,
Step of sending a registration message after storing in the entry/leave management DB if unregistered,
Step of displaying the received registration message on the screen from the customer's terminal,
Step of transmission the inputted leave information from the customer terminal to the control center ,
Step of storing the leave information to the entry/leave management DB from the control center , and
a method for preventing the spread of contagious disease, **characterized in that** the customer terminal terminates the app upon leave completion .

2. In the case of claim 1, the step of the inputting bar code information from the customer's terminal to transmit to the control center for preventing the spread of contagious disease **characterized by** transmitting the mobile phone number entered through the accompanying customer terminal or business s terminal for admission registration of customers without terminals.

3. In the case of claim 2, a customer without a mobile phone number among customers without a terminal for preventing the spread of contagious disease **characterized by** substituting information that can identify an individual such as a social security number ( resident registration number in Korea ) entered instead of a mobile phone number .

4. In the case of claim 1, for preventing the spread of contagious disease, **characterized in that** one or more bar codes can be selected from among 1- and 2 -dimensional bar codes , QR codes , image codes , and loT devices using wireless communication .

5. In case of claim 1, the step of transmitting the inputted leave information to the control center from the customer terminal for preventing the spread of contagious disease **characterized by** transmitting leave information to the control center through the terminal of the establishment instead of the customer's terminal .

6. In case of claim 1, step of transmitting the inputted leave information to the control center from the customer terminal for preventing the spread of contagious diseases, **characterized in that** the customer's terminal compares the location of the customer and the location of the business and automatically transmits the exit information to the control center when there is no input of exit information.

7. In case of claim 1, for checking whether all customers using the establishment have been registered for access , and to request registration if there is an unregistered person;
Step of transmitting a message to request the currently registered customer information from the business terminal;
Step of extracting and transmitting customer information that is currently registered in table units from the control center; and
for preventing the spread of contagious disease, **characterized in that** the terminal of the establishment displays the received information .

8. Unlike establishments managed in table units, in the access records of public transportation such as buses and subways ,
Step of transmitting the code received from the loT device to the control center from the customer's terminal,
Step of transmitting a registration message after storing in the entry /leave management DB if unregistered ,
Step of displaying the received registration message on the screen from the customer terminal,
Step of transmitting leave information to the control center from the customer terminal when there is no information received from the loT device ,
Step of storing the leave information in the entry/leave management DB from the control center , and a method for preventing the spread of contagious diseases, **characterized in that** the customer terminal terminates upon completion of leave.

9. In case of claim 1 , As a method to trace the route of infection ,
Step of transmitting the cell phone number and period of the infected person to the control center from the terminal of the quarantine authorities .
Step of extracting and transmitting information , entry and leave times , table information of all businesses visited during the inquiry period from the control center,
Step of displaying the received information on the screen and transmits the selected business information and inquiry period to the control center in the quarantine authorities.
Step of extracting and transmitting the mobile phone numbers of other customers who have visited the business during the inquiry period, entry/leave time and table information from control center, and
a method for preventing the spread of contagious disease, **characterized in that** the terminal of the quarantine authorities displays the received information on the screen .

10. In case of claim 9 , the actual risk of infection can be evaluated by the distance and contact time from the infected person, and as a means to check the distance in addition to the contact time confirmed by entry and leave,
Step of transmitting a message requesting the establishment's table layout from the quarantine authority's terminal,
Step of extracting and transmitting the table arrangement information of the establishment from the control center, and a method for preventing the spread of contagious disease, **characterized in that** the terminal of the establishment displays the received information.
